Europäisches Patentamt

European Patent Office

Office européen des brevets

(11) Veröffentlichungsnummer: **0 145 939**
A2

(12) **EUROPÄISCHE PATENTANMELDUNG**

(21) Anmeldenummer: **84113455.4**

(51) Int. Cl.⁴: **A 61 F 2/36**

(22) Anmeldetag: **08.11.84**

(30) Priorität: **30.11.83 DE 3343305**

(43) Veröffentlichungstag der Anmeldung: **26.06.85**
**Patentblatt 85/26**

(84) Benannte Vertragsstaaten: **AT BE CH DE FR GB IT LI NL SE**

(71) Anmelder: **Protek AG, Stadtbachstrasse 64, CH-3012 Bern (CH)**

(72) Erfinder: **Weill, Dan, Dr., Château de buy Antilly, F-57640 Vigy (FR)**

(74) Vertreter: **Lusuardi, Werther Giovanni, Dr., Lyss-Strasse 12, CH-3293 Dotzigen (CH)**

(54) **Zementfrei zu implantierender, blattartiger Schaft für eine Hüftgelenkprothese.**

(57) Der blattartige, gerade Schaft (1) verläuft im distalen Bereich auf der lateralen Schmalseite (4) einseitig konisch erweitert; im proximalen Bereich ist die laterale Schmalseite (4) – ebenso wie die mediale (3), sowie der Außenrand des Trochanterflügels (10) – mit konstantem Krümmungsradius nach medial gekrümmt, wobei die Krümmung annähernd dem Calcar-Bogen entspricht.

Die neue Schaftform bewirkt, daß sich der Schaft im distalen Bereich verkeilt und dabei eine resultierende Kraft weitgehend über die ganze Höhe verteilt, von lateral nach medial auf ihn wirkt; diese Kraft gewährleistet eine sichere Auflage der medialen Schmalseite (3) auf dem Calcar-Bogen.

ACTORUM AG

Zementfrei zu implantierender, blattartiger Schaft für
eine Hüftgelenkprothese

Die Erfindung betrifft einen zementfrei zu implantierenden, blattartigen Schaft für eine Hüftgelenkprothese, der
als Geradschaft ausgebildet ist und sich mindestens im
distalen Bereich seiner entlang der Mittelachse gemessenen
Schafthöhe konisch erweitert, und der ferner mit seiner
medialen Schmalseite in einer dem Calcarbogen ähnlichen
Kurve in einen Prothesenhals übergeht, während auf der
gleichsinnig mit der medialen Schmalseite gekrümmten, lateralen Schmalseite ein sich nach lateral erstreckender
und verjüngender Trochanterflügel vorgesehen ist.

Prothesenschäfte der vorstehend genannten Art zur zementfreien Verankerung in einem Röhrenknochen, beispielsweise
Verankerungsschäfte für eine Femurkopfprothese, sind bekannt (siehe z.B. EP-A-0 032 165). Die Verankerung derartiger
Schäfte erfolgt im Femurknochen dadurch, dass einerseits
der Konus im distalen Bereich fest in den Markhohlraum des
Knochens verkeilt ist und andererseits die mediale Schmalseite des Schaftes auf dem Calcar-Bogen aufliegt.

Aufgabe der Erfindung ist es, durch die Formgebung des
neuen Schaftes eine stetige Kraftwirkung in Richtung auf
den Calcar-Bogen auszuüben und so ein Aufliegen und Abstützen
der Prothese auf diesem Bogen zu gewährleisten.

Erfindungsgemäss wird diese Aufgabe dadurch gelöst, dass
die konische Erweiterung des distalen Bereichs auf die laterale Schmalseite beschränkt ist, während die mediale Schmalseite in diesem Bereich parallel zur Mittelachse verläuft.

Durch den einseitigen Konus im distalen Bereich wird dieser Bereich nach medial gedrückt; im proximalen Bereich wird diese medial gerichtete Kraft durch die Formgebung der äusseren Begrenzung des Trochanterflügels erreicht, die in ihrem Verlauf möglichst weitgehend dem Verlauf der "Grenzlinie" zwischen spongiosem und kortikalem Gewebe entspricht, ohne dass sie zwingend entlang dieser "Grenzlinie" verlaufen muss. Darüberhinaus wird die Wirkung der Flügelbegrenzung unterstützt durch das von der Belastung auf den Gelenkkopf ausgeübte Biegemoment. Ein weiterer Vorteil der Formgebung für den Trochanterflügel, der in bekannter Weise als Verdrehsicherung dient, besteht darin, dass die von ihm dabei ausgeübten Torsionskräfte auf den Knochen relativ klein werden.

Die Masse des im Knochengewebe als Fremdkörper wirkenden Schaftes kann relativ auf ein Minimum beschränkt werden, wenn die Seitenflanken der nach lateral gerichteten Verjüngung des Trochanterflügels hohlkehlenartig ausgebildet sind; damit entsteht gleichzeitig beim geradlinigen Einschlagen des Schaftes in Richtung seiner Achse ein Hohlraum, der mit Spongiosa, die beispielsweise dem Femurkopf entnommen wird, gefüllt werden kann.

Für einen festen Sitz einer zementfrei verankerten Prothese ist es wesentlich, dass der Schaft beim Einsetzen und Einschlagen genau geführt wird. Eine solche Führung wird erheblich erleichtert, wenn in die Seitenflanken des Trochanterflügels im Bereich der horizontalen Schulter parallel zur Schaftmittelachse kreiszylindrische Vertiefungen eingearbeitet sind, wobei zusätzlich in den Vertiefungen, in die ein Führungs- und Setzinstrument eingreift, eine Querbohrung für ein Ausziehinstrument vorgesehen sein kann.

Das Aufliegen der medialen Schaftseite auf dem Calcar-Bogen und eine gleichmässige Verteilung der in Richtung auf diesen Bogen wirkenden Kräfte können verbessert werden, wenn die mediale Schmalseite, die laterale Schmalseite und der Trochanterflügel gleiche, annähernd dem Calcar-Bogen entsprechende Krümmungsradien, jedoch unterschiedliche Mittelpunkte haben.

Als Werkstoffe für den neuen Schaft eigenen sich alle in der Implantat-Technik üblichen Materialien; in erster Linie werden die neuen Schäfte aus Metall oder Metall-Legierungen gefertigt.

Im folgenden wird die Erfindung anhand eines Ausführungsbeispiels im Zusammenhang mit der Zeichnung näher erläutert.

  Fig. 1 zeigt eine Aufsicht auf die neue Prothese von anterior oder posterior;

  Fig. 2 ist eine Ansicht von Fig. 1 von links;

  Fig. 3 ist eine Aufsicht auf Fig. 1 von oben, während

  Fig. 4 schliesslich den Schnitt IV-IV von Fig. 1 wiedergibt.

Die laterale Schmalseite 4 des Schaftblattes 1 des neuen Prothesenschaftes erweitert sich von ihrem distalen Ende 9 her konisch bis etwa auf 2/3 der längs der Mittelachse 2 gemessenen Schafthöhe; vom proximalen Ende dieses Konus führt die laterale Schmalseite 4 nach medial gekrümmt in eine horizontale Schulter am proximalen Ende des Schaftes. Diese Schulter geht in einen Prothesenhals 6 über, auf den ein sich nach aussen konisch verjüngender Zapfen 7 aufgesetzt ist, der den nicht gezeigten, kugelförmigen Gelenkkopf aufnimmt.

Die Achse 8 des Zapfens 7 schneidet die Längsmittelachse 2 des Schaftes unter einem Winkel, der im wesentlichen dem Winkel zwischen dem Schenkelhals und der Femurachse eines natürlichen Hüftgelenks entspricht und im vorliegenden Fall etwa $45^{\circ}$ beträgt.

Die mediale Schmalseite 3 läuft zunächst parallel zur Mittelachse 2 und geht anschliessend in einen Bogen über, der in seinem Verlauf weitgehend dem Calcar-Bogen angepasst ist.

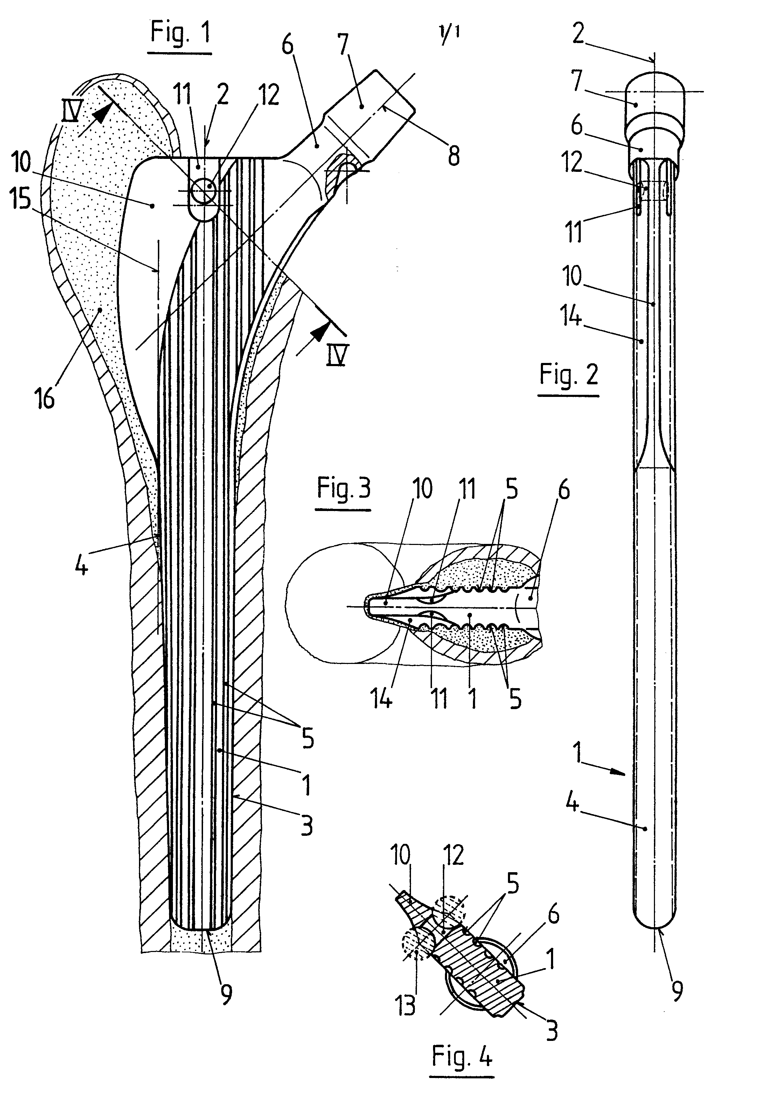

Die Blattseiten des Schaftblattes 1, die, wie Fig. 2 wiedergibt, über die ganze Schafthöhe parallel zueinander sind, sind mit einer aus sich parallel zur Mittelachse 2 erstreckenden Rippen 5 bestehenden Oberflächenstruktur versehen, die das Anwachsen von Knochengewebe fördert.

Aus der lateralen Schmalseite 4 des Schaftblattes 1 springt - etwa zwischen halber und 3/5 der Schafthöhe - in einem zunächst nach lateral gekrümmten Bogen konstanter Krümmung ein Trochanterflügel 10 hervor, dessen Aussenrand anschliessend in einem nach medial gekrümmten Bogen verläuft. Sich nach proximal verbreiternd folgt der Aussenrand des Trochanterflügels 10 im wesentlichen der Krümmung der lateralen Schmalseite 4 und endet ebenfalls in der horizontalen Schulter. In ihm befinden sich Ausnehmungen 11 und eine Bohrung 12 für den Einsatz eines in Fig. 4 angedeuteten Setz- und Führungsinstruments 13 bzw. eines nicht gezeigten Ausziehinstruments.

Bei dem gezeigten Ausführungsbeispiel sind die nach medial gerichteten Krümmungen der beiden Schmalseiten 3 und 4 und des Aussenrands des Trochanterflügels 10 gleich; die sich nach proximal ergebenden Verbreiterungen sind eine Folge unterschiedlich gelegener Mittelpunkte für diese Kreisbögen; der Krümmungsradius der Kreisbögen ist dabei so gewählt, dass er eine möglichst gute Annäherung an einen "Mittelwert" des Calcar-Bogens ergibt.

Wie besonders Fig. 3 und 4 zu entnehmen ist, ist der Trochanterflügel 10 hohlkehlenartig ausgebildet; auf diese Weise entsteht ein Hohlraum 14 (Fig. 3), in den nach Einsetzen der Prothese spongioses Gewebe - beispielsweise aus dem nicht mehr benötigten Femurkopf - eingefüllt und eingestampft werden kann, um die Bildung neuen, den Schaft zusätzlich fixierenden Knochengewebes zu beschleunigen. Die gewählte Formgebung für den Trochanterflügel 10 bringt als weitere Vorteile geringe Substanzverluste an Knochensubstanz, geringe Masse des implantierten Fremdmaterials und eine kontinuierlich abnehmende Festigkeit des Flügels 10 nach lateral. Denn beim Einsetzen des Schaftes 1 muss bei dieser Form des Trochanterflügels 10 lediglich eine etwa rechteckige Operationsöffnung ausgefräst werden, die nach lateral einer parallel zur Mittelachse 2 verlaufenden Tangente 15 (Fig. 1) an den am weitesten lateral gelegenen Punkt der lateralen Schmalseite 4 entspricht, und zusätzlich lediglich ein schmaler Schlitz für den "überstehenden" Bereich des Trochanterflügels 10 im spongiosen Gewebe 16 des Trochanters vorgesehen sein.

Patentansprüche

1. Zementfrei zu implantierender, blattartiger Schaft für eine Hüftgelenkprothese, der als Geradschaft ausgebildet ist und sich mindestens im distalen Bereich seiner entlang der Mittelachse gemessenen Schafthöhe konisch erweitert, und der ferner mit seiner medialen Schmalseite in einer dem Calcarbogen ähnlichen Kurve in einen Prothesenhals übergeht, während auf der gleichsinnig mit der medialen Schmalseite gekrümmten, lateralen Schmalseite ein sich nach lateral erstreckender und verjüngender Trochanterflügel vorgesehen ist, dadurch gekennzeichnet, dass die konische Erweiterung des distalen Bereichs auf die laterale Schmalseite (4) beschränkt ist, während die mediale Schmalseite (3) in diesem Bereich parallel zur Mittelachse (2) verläuft.

2. Schaft nach Anspruch 1, dadurch gekennzeichnet, dass die mediale Schmalseite (3), die laterale Schmalseite (4) und der Trochanterflügel (10) gleiche, annähernd dem Calcarbogen entsprechende Krümmungsradien, jedoch unterschiedliche Mittelpunkte haben.

3. Schaft nach Anspruch 1 oder 2, dadurch gekennzeichnet, dass der Trochanterflügel (10) oberhalb der Mitte, jedoch höchstens bei 3/5 der Schafthöhe, gemessen vom distalen Ende (9), entspringt.

4. Schaft nach einem der Ansprüche 1 bis 3, dadurch gekennzeichnet, dass der Trochanterflügel (10) proximal in eine bezüglich der Mittelachse (2) horizontale Schulter mündet.

5. Schaft nach einem der Ansprüche 1 bis 4, dadurch gekennzeichnet, dass die Seitenflanken der nach lateral gerichteten Verjüngung des Trochanterflügels (10) hohlkehlenartig ausgebildet ist.

6. Schaft nach einem der Ansprüche 1 bis 5, dadurch gekennzeichnet, dass in die Seitenflanken des Trochanterflügels (10) im Bereich der horizontalen Schulter parallel zur Schaftmittelachse (2) kreiszylindrische Vertiefungen (11) eingearbeitet sind.

7. Schaft nach einem der Ansprüche 1 bis 6, dadurch gekennzeichnet, dass in den Vertiefungen (11) eine Querbohrung (12) vorgesehen ist.

8. Schaft nach einem der Ansprüche 1 bis 7, dadurch gekennzeichnet, dass die anterior und posterior gelegenen Blattseiten des Schaftblattes (1) mit einer aus sich parallel zur Mittelachse (2) erstreckenden Rippen (5) bestehenden Oberflächenstruktur versehen sind.

9. Schaft nach einem der Ansprüche 1 bis 8, dadurch gekennzeichnet, dass die anterior und posterior gelegenen Blattseiten des Schaftblattes (1) über die ganze Schafthöhe parallel zueinander sind.

10. Schaft nach einem der Ansprüche 1 bis 9, dadurch gekennzeichnet, dass die Mittelachse (2) einen Winkel von $43^{\circ}$-$47^{\circ}$, vorzugsweise von $44,5^{\circ}$-$45,5^{\circ}$ mit der Achse (8) des Zapfens (7) einschliesst.

0145939

Fig. 1
Fig. 2
Fig. 3
Fig. 4